(19) Europäisches Patentamt / European Patent Office / Office européen des brevets

(11) **EP 3 677 176 A1**

(12) **EUROPEAN PATENT APPLICATION**
published in accordance with Art. 153(4) EPC

(43) Date of publication:
08.07.2020 Bulletin 2020/28

(51) Int Cl.:
*A61B 5/0408* (2006.01)

(21) Application number: 18852070.4

(22) Date of filing: 23.08.2018

(86) International application number:
PCT/JP2018/031121

(87) International publication number:
WO 2019/044649 (07.03.2019 Gazette 2019/10)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.08.2017 JP 2017164065
29.08.2017 JP 2017164066

(71) Applicant: Toyobo Co., Ltd.
Osaka-shi
Osaka 530-8230 (JP)

(72) Inventor: NARUSAWA Haruhiko
Otsu-shi
Shiga 520-0292 (JP)

(74) Representative: Müller-Boré & Partner
Patentanwälte PartG mbB
Friedenheimer Brücke 21
80639 München (DE)

(54) **LIVING-BODY CONTACT ELECTRODE AND CLOTHING FOR MEASURING LIVING-BODY INFORMATION**

(57) The purpose of the present invention is to provide a living-body contact electrode used primarily in a garment for measuring living-body information, which can maintain a comfort at the time of wearing without worrying about rubbing between the electrode and the skin while acquiring a stable electrical signal even in a dry state and a wet state.

A living-body contact electrode can acquire a living-body electrical signal with decreased electrical noises by suppressing the displacement of electrode without impairing feeling and feel of wearing by suppressing the skin irritation by limiting the coefficient of static friction of the surface of the electrode with respect to the skin. By decreasing the frictional coefficient of an electrical wiring part of the present invention to be equal to or less than a predetermined value, and by combining the electrical wiring part with the electrode, an excellent living-body information measurement system capable of trouble-free measurement of living-body information even during exercise can be formed.

**Description**

TECHNICAL FIELD

The present invention relates to a stretchable electrode useful for a garment for measuring living-body information which is excellently pleasant and is brought into contact with the skin surface of a living body to measure a weak electrical signal inside the living body and a garment for measuring living-body information fabricated using the electrode.

BACKGROUND ART

Conventionally, in order to measure weak electrical signals inside a living body such as an electroencephalogram, an electrocardiogram, and an electromyogram, an adhesive pad electrode composed of a conductive adhesive material such as soft conductive solid gel exhibiting conductivity (Patent Document 1), an adsorption electrode composed of a rubber ball and an electrode (Patent Document 2), and the like have been used. These electrodes are based on the premise that an independent electrode is pasted to the skin and measurement is performed when the subject is in a resting state.

Meanwhile, there is a demand for measuring electrical signals over a long period of daily life, and garments to which electrodes are attached are attracting attention. New electrodes to be attached to garments have been devised to replace the adhesive pad electrode to be in danger of protruding or flowing out of gel and the adsorption electrode to be in danger of bulkiness or blood congestion. For example, there is an electrode fabricated by combining a cloth electrode formed by braiding metal conductor fibers such as stainless steel with an electrode formed by imparting impermeability with respect to moisture to a part of the cloth electrode. As impermeability with respect to moisture is imparted, the sheet resistance of the electrode with the skin is lowered by sweating and the influence of electrical noise caused by the cloth-based electrode is avoided (Patent Document 3). According to this method, the impedance between the skin and the electrode depends on sweating, and it is thus concerned that signal acquisition stability is lacking in the case of dry skin and of hardly sweating and the electrode clings or sticks to the skin and the feel of wearing is impaired in the case of sweating.

In addition, in order to avoid mixing of electrical noises which are generated due to displacement of the electrode from the living body when the wearer exercises, a means is taken in which a part of a cloth electrode composed of a conductive fiber structure is equipped with a member having a high coefficient of friction (Patent Document 4). Although it is possible to secure the stability of signal acquisition by making the electrode hardly displace in this manner, attention should be paid to skin injuries and inflammation when the electrode is rubbed with the skin since a part of the electrode is equipped with a member having a high coefficient of friction.

Meanwhile, in order to diminish itching and feeling of wrongness at the time of wearing, there is one in which an electrode equipped with a non-conductive cloth layer on one surface of a conductive cloth knitted with metal-plated fibers is used (Patent Document 5). According to this means, the adhesive property to the skin and the like of the living body is favorable and, at the same time, itching and feeling of wrongness are hardly caused at the time of wearing and the feel of wearing is not impaired. However, it is concerned that the electrical signal is attenuated and the electrical signal cannot be stably acquired since there is an insulating non-conductive layer between the conductive cloth and the skin.

PRIOR ART DOCUMENTS

PATENT DOCUMENTS

    Patent Document 1: JP-A-09-215668
    Patent Document 2: JP-A-11-225977
    Patent Document 3: JP-B-4860155
    Patent Document 4: JP-A-2016-036642
    Patent Document 5: JP-A-2016-182755

SUMMARY OF THE INVENTION

PROBLEMS TO BE SOLVED BY THE INVENTION

In this manner, with regard to the garment to which an electrode is attached and which is used to measure electrical signals over a long period of daily life, it is required to prevent electrical noise and deterioration of electrical signals even when the wearer takes various postures and actions in daily life from a state in which the garment is dry to a state in which the garment is wet with rain and sweat. In addition, the garment is always worn, and thus the electrode is demanded to be stretchable and flexible without being easily displaced while being able to follow the garment when diminishment of the feel of shrink and maintenance of the feel of fitting in the comfort of garment are taken into consideration.

However, in the case of using conventional electrodes to be attached to a garment, there is a problem that the comfort of the garment is sacrificed and attention should be paid to the skin injuries and inflammation due to rubbing of the electrode in order to acquire a stable electrical signal from a dry state to a wet state. An object of the present invention is to provide a living-body contact electrode which can maintain a comfort at the time of wearing without worrying about rubbing between the electrode and the skin while acquiring a stable electrical signal even in a dry state and a wet state and is preferably flexible.

SOLUTIONS TO THE PROBLEMS

The present invention is as follows:

[1] A living-body contact electrode to be used to measure a living-body signal, wherein an area of an electrode surface is 1 square centimeter or more and a coefficient of static friction ($\mu$d) between a surface of the living-body contact electrode and a dry skin is 0.4 or more and 2.0 or less.
[2] The living-body contact electrode according to [1], wherein the coefficient of static friction ($\mu$d) between the surface of the living-body contact electrode and the dry skin is 0.5 or more and 2.0 or less.
[3] A living-body contact electrode to be used to measure a living-body signal, wherein an area of an electrode surface is 1 square centimeter or more and a coefficient of static friction ($\mu$w) between a surface of the living-body contact electrode and a wet skin is 0.3 or more and 3.0 or less.
[4] The living-body contact electrode according to [3], wherein the coefficient of static friction ($\mu$w) between the surface of the living-body contact electrode and the wet skin is 0.4 or more and 3.0 or less.
[5] The living-body contact electrode according to any one of [1] to [4], wherein a stretchable conductor sheet containing at least conductive fine particles and a flexible resin binder as constituent components is used as a conductive member on the surface of the living-body contact electrode.
[6] The living-body contact electrode according to [5], wherein an elastic modulus of the flexible resin binder is 1 GPa or less and a rupture elongation of the flexible resin binder is 200% or more.
[7] The living-body contact electrode according to [5] or [6], wherein the coefficient of static friction ($\mu$d) between the surface of the living-body contact electrode and the dry skin and the coefficient of static friction ($\mu$w) between the surface of the living-body contact electrode and the wet skin satisfy a relational expression below:

$$1.7 \leq (\mu w)/(\mu d) \leq 2.5$$

[8] The living-body contact electrode according to any one of [5] to [7], wherein a water absorption rate of the stretchable conductor sheet is 0.5% by mass or more and 25% by mass or less.
[9] A garment for measuring living-body information, the garment comprising a plurality of the living-body contact electrodes according to any one of [1] to [8].
[10] The garment for measuring living-body information according to [9], wherein a coefficient of static friction ($\mu$d) between an electrical wiring portion surface excluding an electrode surface of a surface in contact with a living body of the garment for measuring living-body information and a dry skin is 0.5 or less.
[11] The garment for measuring living-body information according to [9], wherein a coefficient of static friction ($\mu$d) between an electrical wiring portion surface excluding an electrode surface of a surface in contact with a living body of the garment for measuring living-body information and a wet skin is 0.4 or less.
[12] The living-body contact electrode according to [1] or [2], wherein a conductive fabric is used as a conductive member on the surface of the living-body contact electrode.
[13] The living-body contact electrode according to [3] or [4], wherein a conductive fabric is used as a conductive member on the surface of the living-body contact electrode.
[14] The living-body contact electrode according to [12] or [13], wherein the conductive fabric is formed of a stretchable conductive cloth including at least a conductive fiber as a constituent element.
[15] The living-body contact electrode according to any one of [12] to [14], wherein the conductive fabric is at least one conductive fabric selected from a woven fabric, a knitted fabric, or a nonwoven fabric including a metal-covered synthetic fiber at 10% by mass or more.

[16] The living-body contact electrode according to any one of [12] to [14], wherein the conductive fabric includes a hybrid material of a chemically synthesized fiber and a conductive polymer.

[17] The living-body contact electrode according to any one of [12] to [16], wherein the coefficient of static friction ($\mu$d) between the surface of the living-body contact electrode and the dry skin and the coefficient of static friction ($\mu$w) between the surface of the living-body contact electrode and the wet skin satisfy a relational expression below:

$$0.8 \leq (\mu w)/(\mu d) \leq 1.5$$

[18] The living-body contact electrode according to any one of [12] to [17], wherein a water retention of the conductive fabric is 40% by mass or more and 350% or less.

[19] A garment for measuring living-body information, the garment comprising a plurality of the living-body contact electrodes according to any one of [12] to [18].

[20] The garment for measuring living-body information according to [19], wherein a coefficient of static friction ($\mu$d) between an electrical wiring portion surface excluding an electrode surface of a surface in contact with a living body of the garment for measuring living-body information and a dry skin is 0.5 or less.

[21] The garment for measuring living-body information according to [19], wherein a coefficient of static friction ($\mu$d) between an electrical wiring portion surface excluding an electrode surface of a surface in contact with a living body of the garment for measuring living-body information and a wet skin is 0.4 or less.

Furthermore, the present invention preferably has the following configuration.

[22] The garment for measuring living-body information according to any one of [9], [10], [11], [19], [20], or [21], in which the garment has a means for measuring living-body information of a wearer and a mechanism for transmitting the measured information to the outside.

[23] The garment for measuring living-body information according to any one of [9], [10], [11], [19], [20], or [21], in which the garment has a means for measuring living-body information of a wearer and a mechanism for analyzing the measured information.

[24] The living-body contact electrode according to any one of [5] to [8], in which insulating fine particles are blended at 1% to 10% by volume with respect to a binder resin.

[25] The garment for measuring living-body information according to any one of [9] to [11], in which insulating fine particles are blended at 1% to 10% by volume with respect to a binder resin.

EFFECTS OF THE INVENTION

The present invention has been achieved based on the knowledge that a living-body signal can be favorably acquired as the living-body contact electrode has a predetermined range of coefficient of friction with respect to the skin and the discomfort of wearer is diminished particularly when the difference in coefficient of friction between the dry skin and wet skin is smaller in a living-body contact electrode in which a stretchable conductor sheet containing at least conductive fine particles and a flexible resin binder as constituent components or a conductive fabric is used as a conductive member on the surface of the living-body contact electrode.

According to the configuration of the present invention, the area of the living-body contact electrode which is attached to a part of the inside of garment and is brought into contact with the skin in order to measure a living-body signal is 1 square centimeter or more and the coefficient of static friction ($\mu$d) between the surface of the living-body contact electrode and dry skin is 0.4 or more and 2.0 or less and preferably 0.5 or more and 2.0 or less. By setting the coefficient of static friction to this range, the electrode is less likely to be displaced, the necessary electrode area is secured, a stable electrical signal can be secured, for example, even when the electrode is displaced, and skin injuries and inflammation due to rubbing of the electrode can be prevented in a dry state in which the skin is not sweating. The coefficient of static friction of garment material with the skin is generally about 0.5 at the most, and thus the living-body contact electrode having the coefficient of static friction of the present invention has a small difference in the feel of touch between the garment material and the electrode and can maintain a pleasant feel of wearing. Moreover, as the coefficient of static friction ($\mu$w) between the surface of the living-body contact electrode and the wet skin is 0.3 or more and 3.0 or less, thus the difference in the feel of touch between the garment material and the electrode is small even when the skin is sweating in summer and at the time of exercise and is in a state in which sweat is accumulated between the living-body contact electrode and the skin, and the feel of wearing is not impaired.

Furthermore, the sheet resistance when the living-body contact electrode is not elongated is 300 $\Omega$ $\square$ or less and the sheet resistance increase ratio is less than 10 when the elongation rate of the living-body contact electrode is 10%, thus an electrical signal necessary for measurement is acquired, and a stable electrical signal is secured even when the electrode is elongated along with the garment material which is deformed when the posture changes at the time of wearing. In addition, the tensile modulus of the living-body contact electrode is 1 GPa or less and the load at the time of elongation is 100 N or less when the elongation rate of the living-body contact electrode is 10%, thus the electrode follows the garment material which is deformed when the posture changes at the time of wearing, and feeling of wrongness is thus diminished.

In the present invention, in the case of using a stretchable conductor sheet containing at least conductive fine particles and a flexible resin binder as constituent components as a conductive layer on the surface of the living-body contact electrode, it is preferable that the coefficient of static friction ($\mu$d) between the surface of the living-body contact electrode and dry skin and the coefficient of static friction ($\mu$w) between the surface of the living-body contact electrode and wet skin satisfy the following relational expression.

$$1.7 \le (\mu w)/(\mu d) \le 2.5$$

By this, feeling of wrongness at the time of wearing is diminished both at normal time and at the time of sweating.

In the present invention, in the case of using a stretchable conductor sheet containing at least conductive fine particles and a flexible resin binder as constituent components as a conductive layer on the surface of the living-body contact electrode, the coefficients of friction of the electrode with dry skin and wet skin can be controlled by adjusting the water absorption rate of the stretchable conductor sheet to 0.5% by mass or more and 25% by mass or less. In the present invention, preferably, an electrical signal is secured by the conductive fine particles as conductive particles and insulating fine particles are concurrently used, the coefficient of friction of the electrode surface is adjusted as insulating fine particles are used, these fine particles create voids inside the electrode, water absorbing property is secured, the fine particles are fixed with the binder resin, and the shape as an electrode can be thus maintained. As a binder resin having an elastic modulus of 1 GPa or less and a rupture elongation of 200% or more is used at this time, an electrode exhibiting excellent stretchability is obtained, the electrode follows the garment material, feeling of wrongness is not impaired, and a stable electrical signal is secured even when the electrode is elongated along with the garment material.

In the present invention, in the case of using a conductive fabric as a conductive layer on the surface of the living-body contact electrode, it is preferable that the coefficient of static friction ($\mu$d) between the surface of the living-body contact electrode and dry skin and the coefficient of static friction ($\mu$w) between the surface of the living-body contact electrode and wet skin satisfy the following relational expression.

$$0.8 \le (\mu w)/(\mu d) \le 1.5$$

By this, feeling of wrongness at the time of wearing is diminished both at normal time and at the time of sweating.

In the present invention, in the case of using a conductive fabric as a conductive layer on the surface of the living-body contact electrode, such an effect can be realized by controlling the water retaining property of the conductive fabric. The water retention of the conductive fabric is preferably at least 40% by mass or more, this means that the conductive fabric has both sufficient water supply ability and retention ability, the difference in coefficient of friction due to the degree of skin wetness decreases, and feeling of wrongness of the wearer is diminished. The upper limit of water retention is about 350%.

The living-body contact electrode in the present invention is preferably an electrode which is flexible, can be bent, and can be reversibly elastically deformed by at least 2% or more by pulling in the surface direction.

Incidentally, the coefficient of static friction of the electrode with respect to dry skin or wet skin in the present invention is a coefficient of static friction measured using the bio skin plate to be described later.

MODE FOR CARRYING OUT THE INVENTION

A case in which a stretchable conductor sheet containing at least conductive fine particles and a flexible resin binder as constituent components is used as a conductive member on the surface of a living-body contact electrode will be first described.

The shape of the living-body contact electrode is a sheet shape having an area of 1 square centimeter or more, and the living-body contact electrode is attached to a part of the inside of garment. A stable electrical signal cannot be secured when the electrode is displaced in a case in which the area is less than 1 square centimeter. The living-body contact electrode has a layer structure composed of one or two or more conductive layers, and the electrode material and electrode fabricating method are selected so that the coefficient of static friction ($\mu$d) between the surface of the electrode in contact with the skin and dry skin is 0.4 or more and 2.0 or less and preferably 0.5 or more and 2.0 or less.

By setting the coefficient of static friction ($\mu$d) to 0.4 or more and preferably 0.5 or more, it is possible to prevent the electrode from being easily displaced and to secure a stable electrical signal. In addition, by maintaining the coefficient of static friction ($\mu$d) at 2.0 or less, it is possible to prevent skin injuries and inflammation beforehand without impairing the feel of wearing.

The living-body contact electrode of the present invention may have a stretchable insulating layer on the side facing the garment material. This insulating layer may also serve as an adhesive layer which bonds the garment material with the living-body contact electrode.

In the living-body contact electrode of the present invention, the coefficient of static friction between the surface of the living-body contact electrode and wet skin is 0.3 or more and 3.0 or less and preferably 0.4 or more and 3.0 or less.

In the present invention, in the case of using a stretchable conductor sheet for the contact surface of the living-body contact electrode, the coefficient of static friction of the electrode with wet skin is preferably 1.5 or more and 2.5 or less.

By controlling the coefficient of static friction of the electrode with wet skin in this range, it is possible to prevent skin injuries and inflammation and further to diminish discomfort caused as the electrode clings to the skin as well as to secure a stable electrical signal even when the wearer performs an action accompanied by sweating.

Examples of a method for attaching the living-body contact electrode to garment include a method in which the living-body contact electrode is formed by applying a liquid conductive material (precursor) to the garment material inside the garment and then curing and drying the liquid conductive material, a method in which sewing is performed using a yarn after the living-body contact electrode has been formed in advance, and a method in which the living-body contact electrode is bonded using a liquid bonding agent and a hot melt adhesive sheet.

The conductive fine particles constituting the stretchable conductor sheet of the present invention, preferably the insulating fine particles and the binder resin which are concurrently used will be described.

The conductive fine particles are metal-based fine particles and/or carbon-based fine particles. As the metal-based fine particles, metal particles such as silver, gold, platinum, palladium, copper, nickel, aluminum, zinc, lead, and tin, alloy particles such as brass, bronze, white copper, and solder, hybrid particles such as silver-covered copper, further metal-plated polymer particles, metal-plated glass particles, metal-covered ceramic particles, and the like can be used. As the carbon-based fine particles, graphite powder, activated carbon powder, flaky graphite powder, acetylene black, ketjen black, fullerene, carbon nanotube, and the like can be used. The conductive fine particles may be only one kind or two or more kinds.

Insulating fine particles are fine particles formed of organic or inorganic insulating materials. As the organic fine particles, resin-based fine particles such as acrylic resin fine particles, styrene resin-based fine particles, and melamine resin-based fine particles can be used. As the inorganic fine particles, ceramic-based fine particles such as silica, alumina, zirconia, talc, silicon carbide, magnesia, boron nitride and fine particles of salts, which are hardly soluble in water, such as calcium phosphate, magnesium phosphate, barium sulfate, and calcium sulfate can be used. The insulating fine particles may be only one kind or two or more kinds.

As the binder resin, it is preferable to use a resin having an elastic modulus of 1 GPa or less and a rupture elongation of 200% or more. Examples thereof include thermoplastic resins, thermosetting and photocurable resins, and rubber and elastomers. As the thermoplastic resins, low density polyethylene, ethylene-vinyl acetate copolymer, polyvinylidene chloride, copolymerized polyester, and the like can be used. As the thermosetting and photocurable resins, an acrylic resin, a silicone resin, a polyurethane resin, and the like can be used. Examples of the rubber and elastomers include urethane rubber, acrylic rubber, silicone rubber, butadiene rubber, nitrile rubber, isoprene rubber, styrene-butadiene rubber, butyl rubber, chlorosulfonated polyethylene rubber, ethylene propylene rubber, and vinylidene-fluoride copolymer. The binder resin may be only one kind or two or more kinds.

The living-body contact electrode of the present invention can be obtained by a method in which the living-body contact electrode is obtained by further adding a solvent to conductive fine particles, insulating fine particles, and a binder resin, stirring and kneading these for liquefaction, applying or printing the liquefied mixture on a substrate, then drying the liquefied mixture, and removing the solvent and a method in which the living-body contact electrode is obtained by stirring and kneading conductive fine particles, insulating fine particles, and a liquid binder resin monomer for liquefaction, applying or printing the liquefied mixture on a substrate, then curing the liquefied mixture through heating and light irradiation.

The amount of the conductive fine particles blended is determined in consideration of volume resistivity and stretchability. When the volume percentage is large, the volume resistivity decreases and deterioration of the electrical signal is suppressed but stretchability decreases and the feel of shrink and the feel of fitting are worsened. On the other hand, when the volume percentage is small, the stretchability increases and the feel of shrink and the feel of fitting are improved but the volume resistivity increases and the electrical signal is deteriorated. In order to have a balance between both properties, the amount of conductive fine particles blended with respect to the binder resin is preferably 20% to 60% by volume.

The insulating fine particles can be blended in consideration of the volume resistivity and the coefficient of static friction on the surface of the living-body contact electrode. The addition of insulating fine particles can control the unevenness of the electrode surface and adjust the coefficient of static friction. On the other hand, excessive addition of insulating fine particles leads to the decrease in electrical properties and mechanical properties. In order to have a balance between both properties, the amount of insulating fine particles blended with respect to the binder resin is preferably 1% to 10% by volume. By mainly blending these fine particles, voids are generated inside the electrode and water absorbing property is secured. The water absorption rate of the stretchable conductor sheet constituting the stretchable electrode is preferably 0.5% by mass or more and 25% by mass or less. In a case in which the water absorption rate is less than 0.5% by mass and the electrode surface is flat, sweat absorption is insufficient, the electrode is sticky, and the feel of wearing is insufficient. On the other hand, when the water absorption rate exceeds 25% by mass, there are a large number of voids and the electrode is brittle.

Next, a case in which a conductive fabric is used as a conductive member on the surface of the living-body contact electrode will be described below.

The conductive fabric in the present invention is a conductive fiber structure. As the conductive fabric in the present invention, a fiber structure composed of a fiber material containing at least conductive yarns or fibers can be used. Moreover, in the present invention, the fiber structure obtained by subjecting a non-conductive fiber structure to a treatment for imparting conductivity to can be used.

Examples of the conductive fabric in the present invention include woven fabrics, knitted fabrics, and nonwoven fabrics composed of fibers containing conductive yarns (meaning including conductive yarns and conductive fibers. The same applies hereinafter).

The conductive yarn means a yarn having a resistance value of 100Ω or less per 1 cm of fiber length.

The conductive yarn is a generic term for conductive fibers, bundles of conductive fibers, and twisted yarns, braided yarns, spun yarns, blended yarns which are obtained from fibers including conductive fibers, an ultrafine metal wire obtained by extremely finely drawing a metal wire, and an ultrafine film obtained by cutting a film into an ultrafine fiber shape.

Examples of the conductive fiber in the present invention include chemical fibers or natural fibers covered with metals, chemical fibers or natural fibers covered with conductive metal oxides, chemical fibers or natural fibers covered with carbon-based conductive materials such as graphite, carbon, carbon nanotube, and graphene, chemical fibers or natural fibers covered with conductive polymers.

Moreover, as the conductive fiber, a fiber obtained by spinning a polymer material containing, for example, at least one conductive material selected from the group consisting of metals, conductive metal oxides, carbon-based conductive materials, and conductive polymers can be used.

As the bundle of conductive fibers in the present invention, for example, those obtained by supporting and impregnating a conductive filler, a conductive polymer and the like into bundles formed of microfibers or nanofibers of the above-described conductive fibers can be used.

As the conductive yarn in the present invention, a twisted yarn, a braided yarn, a spun yarn, a blended yarn and the like that are obtained using fibers including the above-described conductive fibers may be used. The conductive yarn includes an ultrafine metal wire obtained by extremely finely drawing a metal wire.

The average diameters of the conductive fibers, the bundles of conductive fibers, the twisted yarns, braided yarns, spun yarns, blended yarns which are obtained from fibers including conductive fibers, and the ultrafine metal wire are preferably 250 $\mu$m or less, more preferably 120 $\mu$m or less, still more preferably 80 $\mu$m or less, and particularly preferably 50 $\mu$m or less.

The conductive yarn also includes an ultrafine film obtained by cutting a film into an ultrafine fiber shape. The ultrafine film means a fibrous film obtained by cutting a polymer film covered with at least one conductive material selected from the group consisting of metals, conductive metal oxides, carbon-based conductive materials, and conductive polymers to have a width of 800 $\mu$m or less.

Among the conductive yarns, it is preferable to use at least one selected from the group consisting of chemical fibers covered with metals, bundles of conductive fiber supported and impregnated with conductive polymers, and ultrafine metal wires having an average diameter of 50 $\mu$m or less.

Specific examples of the conductive fabric in the present invention include a fiber structure in which a conductive yarn is embroidered on a non-conductive cloth, a fiber structure in which a non-conductive cloth is impregnated with a conductive polymer-containing solution and dried, and a fiber structure impregnated with a solution containing a conductive filler and a binder resin and dried. Among these, it is preferable to use a fiber structure in which a non-conductive cloth is impregnated with a conductive polymer-containing solution and dried.

As the conductive polymer, for example, a mixture containing poly(3,4-ethylenedioxythiophene) and polystyrenesulfonic acid can be preferably used. As the fiber containing a conductive yarn, a synthetic fiber multifilament is preferable, and a synthetic fiber multifilament of which at least a part is an ultrafine filament having a fineness of less than 30 dtex or a synthetic fiber multifilament having a fineness of more than 400 dtex and a single yarn fineness of 0.2 dtex or less is preferable.

It is preferable that the conductive fabric is a woven fabric composed of fibers containing conductive yarns, or the basis weight is less than 50 g/m$^2$ for the reason that priority is given to the prevention of falling off of conductive polymer or exceeds 300 g/m$^2$ for the reason that sufficient conductivity is secured in the case of a knitted fabric.

The water retention of the conductive fabric in the present invention is 40% by mass or more and 350% by mass or less. The upper limit of the water retention is preferably 200% by mass and still more preferably 150% by mass. It is not preferable that the water retention is lower than this range since the difference in coefficient of static friction of the living-body contact electrode with respect to dry skin and wet skin is likely to increase. Incidentally, the water retention in the present invention is a method described in JIS L 1913: 2010 Test methods for nonwovens.

In the case of using a conductive fabric, the shape of the living-body contact electrode is a sheet shape having an area of 1 square centimeter or more, and the living-body contact electrode is attached to a part of the inside of garment. A stable electrical signal cannot be secured when the electrode is displaced in a case in which the area is less than 1 square centimeter. The living-body contact electrode has one or plural conductive layers, and the electrode material and electrode fabricating method are selected so that the coefficient of static friction ($\mu$d) between the surface of the conductive layer in contact with the skin and dry skin is 0.4 or more and 2.0 or less, preferably 0.45 or more and 2.0 or less, and still more preferably 0.5 or more and 0.8 or less.

By controlling the coefficient of static friction of the electrode with dry skin in this range, it is possible to prevent the electrode from being easily displaced and to secure a stable electrical signal. In addition, by maintaining the coefficient of static friction ($\mu$d) at 2.0 or less, it is possible to prevent skin injuries and inflammation beforehand without impairing the feel of wearing.

The living-body contact electrode of the present invention may have a stretchable insulating layer on the side facing the garment material. This insulating layer may also serve as an adhesive layer which bonds the garment material with the living-body contact electrode.

In the case of using a conductive fabric, the living-body contact electrode of the present invention is always used by being in contact with the skin in order to measure living-body signals, and thus the coefficient of static friction between the surface of the living-body contact electrode and wet skin is preferably 0.3 or more and 3.0 or less and still more preferably 0.35 or more and 1.5 or less in order to secure a stable electrical signal even when the wearer performs an action accompanied by sweating and to maintain the feel of wearing without causing skin injuries and inflammation. By setting the coefficient of static friction ($\mu$w) to this range, it is possible to prevent electrode displacement and to secure a stable electrical signal. Moreover, by controlling the upper limit of the coefficient of static friction, it is possible to diminish the discomfort that the electrode clings to the skin and to maintain favorable feel of wearing.

The garment for measuring living-body information of the present invention has a configuration in which the living-body contact electrode of the present invention is attached to a part of the inside of garment. The base material of the garment for measuring living-body information of the present invention is not particularly limited as long as it is belt-shaped objects such as a belt and a brassiere and/or apparels composed of a knit fabric and a nonwoven fabric, but those exhibiting stretchability are preferable from the viewpoint of fitting property to the body at the time of wearing and followability at the time of exercise and movement in order to measure living-body information. Such garment for measuring living-body information becomes a means for measuring living-body information of the wearer, has a normal wearing method and normal feel of wearing, and various kinds of living-body information can be conveniently measured by only wearing the garment.

In the garment for measuring living-body information, which is equipped with the living-body contact electrode of the present invention, electrical wiring for connecting a signal acquired from the electrode to the system of measurement is provided. The electrical wiring may be formed using the same material as that of the living-body contact electrode or an arbitrary another conductor, preferably an extensible conductor. Conductive materials to be preferably used in the present invention are metal foils, conductive yarns, stretchable electric wires, conductive fabrics, and the like. As the conductive yarns and conductive fabrics, conductive yarns and conductive fabrics composed of fine metal wires, metal-plated fibers, hybrid materials of fibers and conductive polymers, and the like can be used. In the case of using a metal foil, it is possible to generate pseudo stretchability by processing the metal foil into a zigzag shape or a corrugated shape. Pseudo stretchability can be realized by embroidering the conductive yarn in a zigzag. Moreover, extensibility can also be expressed by disposing the conductive yarn while imparting redundancy through braiding and the like. Such an electrical wiring portion is required to be insulated for safety, leakage prevention, and maintenance of signal quality. Such insulation is electric wire covering in the case of a stretchable electric wire. In the metal foil, conductive yarn, and conductive fabric, an insulating coat exhibiting flexibility and stretchability is preferably used.

In the present invention, as a method for attaching the living-body contact electrode to a garment, a method in which the living-body contact electrode is formed in advance and then sewed using a yarn, a method in which the living-body contact electrode is bonded using a liquid bonding agent and a hot melt adhesive sheet, a method in which the conductive yarn is directly sewed and attached to the garment material, a method in which the conductive yarn is partially knitted to constitute garment, and the like can be used.

As the electrical properties of the living-body contact electrode of the present invention, the sheet resistance when the living-body contact electrode is not elongated is 300 $\Omega\square$ or less to have a low sheet resistance that is sufficient to measure weak living-body signals and the sheet resistance increase ratio is less than 10 at an elongation rate of 10% to acquire stable electrical signals even when the electrode is elongated along with the garment material at the time of wearing. Stable electrical signals cannot be acquired in a case in which the sheet resistance when the living-body contact electrode is not elongated exceeds 300 Ω □ and the sheet resistance increase ratio is 10 or more at elongation rate of 10%.

As the elongation property of the living-body contact electrode of the present invention, the tensile modulus is 1 GPa or less and the load at the time of elongation is 100 N or less when the elongation rate of the living-body contact electrode is 10%, thus the electrode is elongated to follow the garment material even when the garment material is elongated and the feel of wearing is not impaired when the living-body contact electrode is used by being attached to garment.

In a case in which the living-body contact electrode of the present invention is applied to a garment material of garment for measuring living-body information, it may be required to form an insulating layer. The insulating layer can be composed of a flexible resin coating or a flexible resin sheet laminate. As the flexible resin which can be used as the insulating layer of the present invention, it is preferable to use a resin having an elastic modulus of 1 GPa or less and a rupture elongation of 200% or more, and examples thereof include thermoplastic resins, thermosetting/photocurable resins, and rubber and elastomers. As the thermoplastic resins, low density polyethylene, ethylene-vinyl acetate copolymer, polyvinylidene chloride, copolymerized polyester, and the like can be used. As the thermosetting and photocurable resins, an acrylic resin, a silicone resin, a polyurethane resin, and the like can be used. Examples of the rubber and elastomers include urethane rubber, acrylic rubber, silicone rubber, butadiene rubber, nitrile rubber, isoprene rubber, styrene-butadiene rubber, butyl rubber, chlorosulfonated polyethylene rubber, ethylene propylene rubber, and vinylidene-fluoride copolymer. The binder resin may be only one kind or two or more kinds.

In the garment for measuring living-body information, which is equipped with the living-body contact electrode in the present invention, the coefficient of static friction ($\mu$d) between the electrical wiring portion surface excluding the electrode surface of the surface in contact with a living body of the garment for measuring living-body information and dry skin is preferably 0.50 or less, more preferably 0.40 or less, and still more preferably 0.25 or less regardless of the raw material of the contact surface. Incidentally, the electrical wiring portion surface herein means the surface of the insulating film in a case in which the electrical wiring is accompanied by insulation.

In the garment for measuring living-body information of the present invention, the coefficient of static friction ($\mu$d) between the electrical wiring portion surface excluding the electrode surface of the surface in contact with a living body of the garment for measuring living-body information and wet skin is preferably 0.4 or less and still more preferably 0.32 or less regardless of the raw material of the contact surface. The coefficient of static friction is yet more preferably 0.24 or less.

When the coefficient of friction of the electrical wiring portion with respect to dry skin or wet skin exceeds this range, the discomfort that the subject feels at the time of wearing increases. It is not preferable that the wearer feels discomfort since the living-body information may be affected by the discomfort.

EXAMPLES

Next, specific Examples of the present invention will be described, but the present invention is not particularly limited to these Examples.

Examples of a case in which a stretchable conductor sheet containing at least conductive fine particles and a flexible resin binder as constituent components is used as a conductive member on the surface of a living-body contact electrode will be first described.

[Preparation of conductive paste]

The materials presented in Table 1 were used, and the resin was dissolved in each solvent at the weight blending ratio presented in Table 2, and silver particles were blended into the solution obtained, and the mixture was mixed in a three-roll mill to obtain a conductive paste.

[Table 1]

| Material | Abbreviation | Contents |
|---|---|---|
| Resin | CSM | Chlorosulfonated polyethylene rubber CSM-TS530 manufactured by TOSOH CORPORATION |
| | DN3 | Nitrile rubber Nipol DN003 manufactured by ZEON CORPORATION |
| | UR6 | Polyester urethane resin VYLON UR6100 manufactured by TOYOBO CO., LTD. |

(continued)

| Material | Abbreviation | Contents |
|---|---|---|
| Solvent | CHX | Cyclohexanone |
| | IPH | Isophorone |
| | SOL | Solvesso |
| Conductive fine particles | G35 | Aggregated silver powder G-35 manufactured by DOWA ELECTRONICS MATERIALS CO.. LTD. |
| | FA3 | Flaky silver powder FA-D-3 manufactured by DOWA ELECTRONICS MATERIALS CO.. LTD. |
| Insulating fine particles | TS1 | Precipitated barium sulfate TS-1 manufactured by TAKEHARA KOGYO CO., LTD. |
| | 350 | Silica fine powder SILYSIA 350 manufactured by FUJI SILYSIA CHEMICAL LTD. |
| Hot melt sheet | UH | 'Polyurethane hot melt ELPHAN UH manufactured by Nihon Matai Co., Ltd. |
| Shirt | Polyester | Cool One 100% polyester manufactured by SANWA CO., LTD. |

[Fabrication of living-body contact electrode]

The conductive paste was applied on a release-treated PET film using an applicator so that the dry film thickness was about 100 μm and dried in a hot air drying oven at 120°C for 30 minutes, and then the release-treated PET film was peeled off to obtain a sheet-shaped living-body contact electrode. The thickness, sheet resistance when the living-body contact electrode was not elongated, sheet resistance increase ratio when the living-body contact electrode was elongated, and load when the living-body contact electrode was elongated were measured using this sheet by the methods to be described below. The measurement results of Examples are presented in Table 2.

[Fabrication of garment equipped with electrode and wiring]

The hot melt sheet and release paper presented in Table 1 were superimposed on the living-body contact electrode with release-treated PET film fabricated above, and these were bonded to each other using a roll laminator of which the rubber roll temperature was adjusted to 120°C to obtain an adhesive living-body contact electrode sheet. This living-body contact electrode sheet was cut into a 5.0 cm square shape following a wiring width of 1.0 cm and a wiring length of 15.0 cm. The release paper was peeled off from the cut-out electrode with wiring, the electrode was disposed at a predetermined position on the back side of a shirt (100% polyester) and bonded thereto by thermocompression using an iron, and the release-treated PET film was further peeled off to fabricate a shirt equipped with an electrode on the back side.

[Fabrication of shirt incorporated with heart rate measuring function]

A stainless steel hook was attached to the front side of the wiring end of the shirt equipped with electrode and wiring so as to conduct with the wiring on the back side, and a heart rate sensor WHS-2 manufactured by UNION TOOL CO. was connected to the electrode via the stainless steel hook to fabricate a shirt incorporated with a heart rate measuring function.

[Elongation test and measurement of sheet resistance]

Using an elongation testing machine (hand drawing stretcher) equipped with two 2.5 cm wide chucks, the stretchable conductive material sheet fabricated above was sandwiched between the chucks so that the distance between the chucks was 5.0 cm and elongated (displacement magnitude: 0.5 cm) in the longitudinal direction to an elongation rate of 10%. As the sheet resistance before and after the test, the resistance values (Ω) before and after elongation on the outside (measurement distance: 10 cm) of the two facing chucks were measured using a digital multimeter ("YOKOGAWA TY530" manufactured by Yokogawa Test & Measurement Corporation) to attain the sheet resistance (Ω □) of the living-body contact electrode. The resistance value was measured immediately after elongation (within 3 seconds).

[Measurement of load at time of elongation]

The load (N) applied when a stretchable conductive material sheet having a width of 30 mm and a test length of 50 mm was elongated to an elongation rate of 10% was measured using a tensile testing machine ("RTM-250" manufactured by ORIENTEC CORPORATION) to attain the unit load (N/cm) per 1 cm sheet length.

[Measurement of coefficient of static friction]

The coefficients of static friction of the living-body contact electrode with respect to the pseudo skin when the skin was dry and wet were measured using a static friction coefficient measuring machine (TriboGear TYPE: 10 manufactured by SHINTO Scientific Co., Ltd.) and determined as follows. As the pseudo skin used in the evaluation, a bio skin plate (product number: P001-001, length 195mm × width 120mm × thickness 5mm manufactured by Burrluck Co., Ltd.) was used in which the surface of the urethane elastomer film was processed to reproduce hydrophilicity and hydrophobicity close to the human skin and surface wrinkles. As the coefficient of static friction ($\mu$d) when the skin was dry, the bio skin plate was fixed to the rising plate in the horizontal state, and the living-body contact electrode was attached to a flat indenter of 75 mm length × 35 mm width, was left to stand for 30 seconds under the condition of 150 g load, and then inclined at an average rising speed of 10 degrees/6 seconds, and the friction angle ($\theta$d, degree) when the flat indenter started to slide was read to attain pd = tan ($\theta$d × $\pi$/180). As the coefficient of static friction ($\mu$w) when the skin was wet, the living-body contact electrode was attached to a flat indenter, then 1 ml of water was applied to the electrode, and the friction angle ($\theta$w, degree) was read by the same operation as that in the measurement when the skin was dry to attain $\mu$w = tan ($\theta$w × $\Pi$/180). The measurement results of Examples and Comparative Examples are presented in Table 2.

[Measurement of water absorption rate]

The water absorption rate was measured in conformity with JIS L1907 Testing methods for water absorbency of textiles, 7.2 water absorption rate method. Three 75 mm × 75 mm test pieces were sampled from the living-body contact electrode sample, and the mass (Wd, mg) thereof was measured, then the test pieces were submerged in an immersion tank containing ion-exchanged water and immersed for 20 minutes. After being immersed, the test pieces were taken out from the immersion tank, sandwiched between two sheets of 200 mm × 200 mm square filter paper, and then pressed for 3 seconds using a 6.75 kg weight with a base of 100 mm × 100 mm. The mass (Ww, mg) of the test piece was immediately measured, and the water absorption rate (c, % by mass) was determined by the following Formula (1).

$$c = (Ww - Wd)/Wd \times 100 \qquad (1)$$

[Feel of wearing and SN ratio in electrocardiogram measurement]

Five adult men were selected as the subject, wore the shirt which was incorporated with a heart rate measuring function and fabricated in Examples, and continuously performed radio calisthenics No. 1 and radio calisthenics No. 2 while the electrocardiogram was measured in an environment of 15°C and 40% RH. With regard to the feel of wearing during that time, five-step sensory evaluation was performed by granting 5 points to "favorable feel of touch" and 1 point to "poor feel of touch", and it was judged as ◎ when the average of the 5 people was 4 or more, ○ when the average was 3 or more and less than 4, △ when the average was 2 or more and less than 3, and × when the average was less than 2.

As the SN ratio at the time of electrocardiogram (ECG) measurement, the SN ratio was determined by the formula of S/N where the variance of the amplitude of the R wave from the ECG waveform excluding the first one minute and the last one minute of the calisthenics time was taken as the signal (S) and the variance of the amplitude of the waveform between the R wave and R wave was taken as the noise (N). The measurement results of Examples and Comparative Examples are presented in Table 2.

[Table 2]

| Item | | Example 1 | Example 2 | Example 3 | Example 4 | Comparative Example 1 | Comparative Example 2 |
|---|---|---|---|---|---|---|---|
| Resin (parts by mass) | CSM | 19 | - | - | - | 30 | - |
| | DN3 | - | 18 | 20 | - | - | 15 |
| | UR6 | - | - | - | 20 | - | - |
| Solvent (parts by mass) | CHX | - | - | - | 15 | - | - |
| | IPH | - | 45 | 45 | 10 | - | 45 |
| | SOL | 45 | - | - | 20 | 35 | - |
| Conductive fine particles (parts by mass) | G35 | 78 | 58 | 77 | 58 | 50 | 57 |
| | FA3 | - | 20 | - | 20 | - | 20 |
| Insulating fine particles (parts by mass) | TS1 | 3 | 4 | - | - | - | - |
| | 350 | - | - | 3 | 2 | - | 8 |
| Thickness ($\mu$ m) | | 94 | 98 | 97 | 103 | 101 | 96 |
| Sheet resistance ($\Omega\ \square$) | | 124 | 72 | 118 | 55 | 208 | 181 |
| Sheet resistance increase rate at 10% elongation | | 2.1 | 1.6 | 1.8 | 1.7 | 11.2 | 7.2 |
| Unit load at 10% elongation (N/cm) | | 38 | 55 | 43 | 64 | 22 | 128 |
| Coefficient of static friction in dry state ($\mu$ d) | | 1.1 | 0.9 | 0.9 | 0.8 | 2.2 | 0.3 |
| Coefficient of static friction in wet state ($\mu$ w) | | 2.0 | 1.7 | 1.6 | 1.7 | 3.4 | 0.4 |
| Dry/wet ratio $\mu$ w/p d | | 1.82 | 1.89 | 1.78 | 2.13 | 1.55 | 1.33 |
| Water absorption of electrode sheet (%) | | 6.3 | 3.9 | 5.1 | 2.9 | 0.7 | 14.5 |
| Feel of wearing | | ○ | ○ | ○ | ○ | × | ○ |
| SN ratio | | 3.8 | 3.6 | 4.3 | 3.9 | 4.4 | 1.2 |

<Application Example 1>

A pair of die-cut component sheets obtained by integrating the living-body contact electrode part, the electrical wiring portion, and the connector portion were fabricated using the sheet-shaped living-body contact electrode obtained in Example 1. Subsequently, the pair of die-cut component sheets were disposed at the left and right chest portions of a sports shirt made of a garment material knitted using a polyester-cotton-polyurethane blended yarn so that the electrode-disposed portion was located about 10 cm below the nipple equivalent position, and laminated using an iron. Furthermore, the product name "Mobilon" (manufactured by Nisshinbo Holdings Inc.), which was a urethane sheet with hot melt layer, used as an insulating layer was pasted to the electrical wiring portion so as to cover the electrical wiring portion using an iron. The coefficient of friction between the surface (insulating layer covered the electrical wiring) of the electrical wiring portion and the bio skin plate when the skin was dry was 0.36, and the coefficient of friction when the skin was wet was 0.24. A snap hook was sewed with a conductive yarn to the connector portion corresponding to the opposite end of the living-body contact electrode of the electrical wiring to form a connector, and a garment for measuring living-body information was thus obtained.

A heart rate sensor WHS-2 manufactured by UNION TOOL CO., which had a wireless data transmission function to a smartphone, was connected to the garment for measuring living-body information obtained as a removable electronic unit, and it was set so that an ECG signal was sent from the WHS-2 to the smartphone simultaneously with the measurement, the heart rate data was received with a smartphone manufactured by Apple Inc. equipped with the app "myBeat" dedicated to the heart rate sensor WHS-2 and displayed on the screen, and further RRI (ms (milliseconds)) and HR (bpm (heart rate/minute)) was able to be measured. Incidentally, RRI is the time interval between the R wave, which is the wave with the largest potential difference in ECG, and the next R wave, and (heart rate) = 60/(R - R time (seconds)) [bpm] can be calculated from RRI.

An electrocardiogram information measuring system using garment for measuring living-body information having a heart rate measuring function was configured in a manner as described above. Five subjects wore garment for measuring living-body information obtained and continuously performed radio calisthenics No. 1 and radio calisthenics No. 2 while the electrocardiogram was measured. As a result, the electrocardiogram measurement of all five subjects during the calisthenics was able to be performed without any problems. In addition, none of the five subjects particularly complained of feeling of wrongness or discomfort.

<Application Example 2>

The sheet-shaped living-body contact electrode obtained in Example 2 was punched into a shape having a major axis of 40 mm and a minor axis of 20 mm to form an electrode portion. Subsequently, the electrode portion was pasted to almost equivalent positions as in Application Example 1 of the same sports shirt as in Application Example 1 using an iron, further a conductive yarn (silver-coated yarn) was embroidered from the left and right electrode portions toward the chest center of the sports shirt in a zigzag to form electrical wiring portions, and a snap hook for connection was sewed at a predetermined position with a conductive yarn to form a connector. Furthermore, the same garment material as that of the sports shirt was sewed so as to cover the inside of garment at the part on which the silver-coat yarn was embroidered to form an insulating cover, and garment for measuring living-body information was thus obtained. Incidentally, the coefficient of friction between the surface (insulating cover covered the electrical wiring) of the electrical wiring portion and the bio skin plate when the skin was dry was 0.16, and the coefficient of friction when the skin was wet was 0.23.

Hereinafter, a system having a heart rate measuring function was configured in the same manner as in Application Example 1, and five subjects wore the system and continuously performed radio calisthenics No. 1 and radio calisthenics No. 2 while the electrocardiogram was measured. As a result, the electrocardiogram measurement of all five subjects during the calisthenics was able to be performed without any problems. In addition, none of the five subjects particularly complained of feeling of wrongness or discomfort.

<Application Comparative Example>

In Application Example 2, an insulating layer was formed using a silicone resin caulking agent instead of the same garment material as that of the sports shirt as the insulating cover. The coefficient of friction of the electrical wiring portion having the insulating cover layer of the silicone resin caulking agent with the bio skin plate when the skin was dry was 0.56 and the coefficient of friction when the skin was wet was 0.42.

Hereinafter, a system having a heart rate measuring function was configured in the same manner as in Application Example 1, and five subjects wore the system and continuously performed radio calisthenics No. 1 and radio calisthenics No. 2 while the electrocardiogram was measured. As a result, the noises increased from the second half of radio calisthenics No. 2 and the electrocardiogram was not able to be measured in two who had a relatively large sweat rate among the five subjects. In addition, the two also complained that they felt the sense that the sports shirt was gradually pulled as they sweated.

Next, Examples of a case in which a conductive fabric is used as a conductive member on the surface of the living-body contact electrode will be described below.

<Production Example 1 of conductive yarn>

A nylon fiber twisted yarn (250 denier) was plated with silver by electroless plating. First, as a ground treatment for electroless silver plating, the twisted yarn was immersed in a scour preparation, washed with water, then immersed in an aqueous solution containing stannous chloride at 10 g/liter and 35% hydrochloric acid at 20 ml/liter, then washed with water to impart catalytic property to the twisted yarn, and then covered with silver by 10% by mass using a predetermined amount of electroless silver plating solution having the following composition.

[Electroless silver plating solution bath ratio] (silver 5 g/1 L)

Tetrasodium ethylenediaminetetraacetate 100 g/1 liter

Sodium hydroxide 25 g/1 liter
Formalin 50 g/1 liter
Silver nitrate (dissolved in 1 liter of water and added dropwise) 15.8 g
Ammonia water (dissolved in 1 liter of water and added dropwise) 50 ml

The silver-plated yarn obtained was twisted to obtain a conductive yarn (F1) having a resistance value of 120 m$\Omega$ per 1 cm of yarn length.

<Production Example 2 of conductive yarn>

An aramid fiber covered with silver by 20% by weight was obtained using an aramid fiber in the same manner as in Production Example 1 of conductive yarn except that the amount of fiber processed per electroless silver plating bath was decreased by half. The fiber obtained was twisted to obtain a conductive yarn (F2) having a resistance value of 65 m$\Omega$ per 1 cm of yarn length.

<Production Example 3 of conductive yarn>

A polyester multifilament processed yarn of 50 denier 24 filament was subjected to 10% weight loss processing by alkaline hydrolysis, thoroughly washed with water, and subsequently immersed in an aqueous solution containing palladium chloride at 0.3 g/L, stannous chloride at 30 g/L, and 36% hydrochloric acid at 300 ml/L at 40°C, and then washed with water. Subsequently, the resultant yarn was immersed in borohydrofluoric acid having an acid concentration of 0.1 N and then washed with water. Next, the resultant yarn was immersed in an electroless copper plating solution containing copper sulfate at 7.5 g/L, 37% formalin at 30 ml/L, and Rochelle salt at 85 g/L and then washed with water. Subsequently, the resultant yarn was immersed in an electric nickel plating solution which contained nickel sulfamate at 300 g/L, boric acid at 30 g/L, and nickel chloride at 15 g/L and had a pH 3.7 while being energized to obtain a conductive yarn (F3) having a copper/nickel two-layer metal covering layer. The metal content in the conductive yarn obtained was 8% by mass for copper and 2% by mass for nickel in terms of mass. Moreover, the resistance value per 1 cm of yarn length was 300 m$\Omega$.

<Production Example 4 of conductive yarn>

A spun yarn of ultrafine fiber Belima X manufactured by KB SEIREN, LTD. was immersed in a conductive paint Denatron used for conductive components using thiophene-based conductive polymer PEDOT: PSS, subjected to an ultrasonic treatment for 3 minutes, then pulled up while being drawn, and dried to obtain a conductive yarn (F4) impregnated with a conductive polymer. The resistance value of the conductive yarn obtained per 1 cm yarn length was 40 $\Omega$.

<Production Example 5 of conductive yarn>

Nineteen strands of copper-silver alloy (diameter: 80 $\mu$m) manufactured by SWCC SHOWA CABLE SYSTEMS CO., LTD. were twisted to obtain a conductive yarn (F5). The resistance value of the conductive yarn F5 obtained per 1 cm was 0.026 m$\Omega$.

<Production Example 6 of conductive yarn>

A conductive yarn CLACARBO KC-792R-B22T4 manufactured by KURARAY TRADING Co., Ltd. in which heat resistant nylon was used as the core fiber and the surface was covered with carbon-containing nylon 6 resin was used as a conductive yarn (F6). The resistance value of the conductive yarn F6 per 1 cm was 500 k$\Omega$. Incidentally, the resistance value of the present conductive yarn was measured using a normal tester.

[Preparation of conductive fabric and evaluation of living-body contact electrode]

Conductive fabrics presented in Table 3 were obtained by mixing the conductive yarns obtained in Production Examples 1 to 6 with polyester fibers and knitting the mixture into knit. The evaluation results of the living-body contact electrodes fabricated using the respective conductive fabrics are presented in Table 3.

[Table 3]

| | Example 5 | Example 6 | Example 7 | Example 8 | Comparative Example 3 | Comparative Example 4 |
|---|---|---|---|---|---|---|
| Conductive yarn production example | 1 | 2 | 3 | 4 | 5 | 6 |
| Weaving and knitting texture | nonwoven | nonwoven | nonwoven | nonwoven | nonwoven | knit |
| Sheet resistance [Ω □] | 12 | 8 | 45 | 270 | 3.5 | 520 |
| Sheet resistance increase rate at 10% elongation | 3.2 | 1.5 | 0.4 | 0.2 | 0 | 2.5 |
| Tensile modulus [Mpa] | 260 | 410 | 250 | 760 | 600 | 120 |
| Load at 10% elongation [N] | 35 | 46 | 52 | 28 | 120 | 80 |
| Coefficient of static friction with dry skin ($\mu$ d) | 0.54 | 0.53 | 0.46 | 0.48 | 0.49 | 0.31 |
| Coefficient of static friction with wet skin ($\mu$ w) | 0.63 | 0.43 | 0.38 | 0.42 | 0.31 | 0.27 |
| Dry/wet ratio $\mu$ w/$\mu$ d | 1.17 | 0.81 | 0.83 | 0.88 | 0.63 | 0.87 |
| Water retention [mass%] | 72 | 63 | 55 | 105 | 8.5 | 25 |
| Feel of wearing | ○ | ○ | ○ | ○ | × | ○ |
| Possibility of ECG measurement | Possible | Possible | Possible | Possible | Possible | Impossible |
| SN ratio | 3.8 | 3.6 | 4.3 | 3.9 | 4.4 | - |

Incidentally, the property values in Table 3 were measured by the following methods.

[Elongation test and measurement of sheet resistance]

Using an elongation testing machine (hand drawing stretcher) equipped with two 2.5 cm wide chucks, the stretchable conductive material sheet fabricated above was sandwiched between the chucks so that the distance between the chucks was 5.0 cm and elongated (displacement magnitude: 0.5 cm) in the longitudinal direction to an elongation rate of 10%. As the sheet resistance before and after the test, the resistance values ($\Omega$) before and after elongation on the outside (measurement distance: 10 cm) of the two facing chucks were measured using a digital multimeter ("YOKOGAWA TY530" manufactured by Yokogawa Test & Measurement Corporation) to attain the sheet resistance ($\Omega$ □) of the living-body contact electrode. The resistance value was measured immediately after elongation (within 3 seconds).

[Measurement of load at time of elongation]

The load (N) applied, when a stretchable conductive material sheet having a width of 30 mm and a test length of 50 mm was elongated to an elongation rate of 10%, was measured using a tensile testing machine ("RTM-250" manufactured by ORIENTEC CORPORATION) to attain the unit load (N/cm) per 1 cm sheet length.

[Measurement of coefficient of friction]

The coefficients of static friction of the living-body contact electrode with respect to the pseudo skin when the skin was dry and wet were measured using a friction coefficient measuring machine (TriboGear TYPE: 10 manufactured by Shinto Scientific Co., Ltd.) and determined as follows. As the pseudo skin used in the evaluation, a bio skin plate (product number: P001-001, length 195mm × width 120mm × thickness 5mm manufactured by Burrluck Co., Ltd.) was used in which the surface of the urethane elastomer film was processed to reproduce hydrophilicity and hydrophobicity close to the human skin and surface wrinkles. As the coefficient of static friction ($\mu$d) when the skin was dry, the bio skin plate was fixed to the rising plate in the horizontal state, and the living-body contact electrode was attached to a flat indenter of 75 mm length × 35 mm width, was left to stand for 30 seconds under the condition of 150 g load, and then inclined at an average rising speed of 10 degrees/6 seconds, and the friction angle ($\theta$d, degree) when the flat indenter started to slide was read to attain pd = tan ($\theta$d × $\pi$/180). As the coefficient of static friction ($\mu$w) when the skin was wet, the living-body contact electrode was attached to a flat indenter, then 1 ml of water was applied to the electrode, and the friction angle ($\theta$w, degree) was read by the same operation as that in the measurement when the skin was dry to attain pw = tan ($\theta$w × $\pi$/180).

[Measurement of water retention]

The water retention was measured by a method conforming to JIS L 1913: 2010 Test methods for nonwovens.

[Fabrication of living-body contact electrode sheet]

The conductive fabric obtained was temporarily bonded onto a release-treated PET film, a hot melt sheet and release paper were further superimposed thereon, and these were bonded to each other using a roll laminator of which the rubber roll temperature was adjusted to 120°C to obtain a hot melt adhesive living-body contact electrode sheet. This living-body contact electrode sheet was cut into a rectangle having a wiring width of 1.0 cm and a wiring length of 15.0 cm and a circle which had a diameter of 5.0 cm and was directly connected to the rectangle. The circular portion corresponds to the electrode, and the rectangular portion corresponds to the wiring portion. The cut-out living-body contact electrode sheet was peeled off from the release-treated PET film, disposed at a predetermined position on the back side of a shirt (100% polyester) and bonded thereto by thermocompression using an iron, a hot melt sheet was further superimposed on the wiring portion and pressure-bonded to form an insulating layer, and a shirt equipped with an electrode and wiring on the back side was thus fabricated.

[Fabrication of shirt incorporated with heart rate measuring function]

A stainless steel hook was attached to the front side of the wiring end of the shirt equipped with an electrode and wiring obtained so as to conduct with the wiring on the back side, and a heart rate sensor WHS-2 manufactured by UNION TOOL CO. was connected to the electrode via the stainless steel hook to fabricate a shirt incorporated with a heart rate measuring function.

[Feel of wearing and SN ratio in electrocardiogram measurement]

Five adult men were selected as the subject, wore the shirt which was incorporated with a heart rate measuring function and fabricated in Examples, and continuously performed radio calisthenics No. 1 and radio calisthenics No. 2 while the electrocardiogram was measured in an environment of 15°C and 40% RH. With regard to the feel of wearing during that time, five-step sensory evaluation was performed by granting 5 points to "favorable feel of touch" and 1 point to "poor feel of touch", and it was judged as ◎ when the average of the 5 people was 4 or more, ○ when the average was 3 or more and less than 4, △ when the average was 2 or more and less than 3, and × when the average was less than 2.

As the SN ratio at the time of electrocardiogram (ECG) measurement, the SN ratio was determined by the formula of S/N where the variance of the amplitude of the R wave from the ECG waveform excluding the first one minute and the last one minute of the calisthenics time was taken as the signal (S) and the variance of the amplitude of the waveform between the R wave and R wave was taken as the noise (N). The evaluation of garment for measuring living-body information which was equipped with an electrode and wiring and fabricated using a conductive fabric fabricated using a conductive yarn obtained in the same manner was performed below. The results are presented in Table 3.

As described above, the living-body contact electrode of the present invention can acquire a living-body electrical signal with decreased electrical noises by suppressing the displacement of electrode without impairing feeling and feel of wearing by suppressing the skin irritation by limiting the coefficient of static friction of the surface of the electrode with respect to the skin. Moreover, as presented in Application Examples, the garment for measuring living-body information having the configuration of the present invention can be usefully used as an input interface of the living-body information measuring system as can be seen from the fact that ECG can be measured at the time of exercise.

INDUSTRIAL APPLICABILITY

The present invention provides a living-body contact electrode and a garment for measuring living-body information, which enable favorable measurement without impairing feeling and feel of wearing even under dry conditions in winter and high humidity conditions in summer. These can be applied to health management in daily life, grasping living-body information during outdoor sports such as jogging and marathons, and labor management in outdoor work at construction sites. In addition, in the present specification, for the sake of convenience, the present invention has been described on the assumption that the electrode and garment are worn by humans but is not limited to the electrode and garment for humans and can be widely used for pets, livestock, and wild animals.

**Claims**

1. A living-body contact electrode to be used to measure a living-body signal, wherein an area of an electrode surface is 1 square centimeter or more and a coefficient of static friction ($\mu$d) between a surface of the living-body contact electrode and a dry skin is 0.4 or more and 2.0 or less.

2. The living-body contact electrode according to claim 1, wherein the coefficient of static friction ($\mu$d) between the surface of the living-body contact electrode and the dry skin is 0.5 or more and 2.0 or less.

3. A living-body contact electrode to be used to measure a living-body signal, wherein an area of an electrode surface is 1 square centimeter or more and a coefficient of static friction ($\mu$w) between a surface of the living-body contact electrode and a wet skin is 0.3 or more and 3.0 or less.

4. The living-body contact electrode according to claim 3, wherein the coefficient of static friction ($\mu$w) between the surface of the living-body contact electrode and the wet skin is 0.4 or more and 3.0 or less.

5. The living-body contact electrode according to any one of claims 1 to 4, wherein a stretchable conductor sheet containing at least conductive fine particles and a flexible resin binder as constituent components is used as a conductive member on the surface of the living-body contact electrode.

6. The living-body contact electrode according to claim 5, wherein an elastic modulus of the flexible resin binder is 1 GPa or less and a rupture elongation of the flexible resin binder is 200% or more.

7. The living-body contact electrode according to claim 5 or 6, wherein the coefficient of static friction ($\mu$d) between the surface of the living-body contact electrode and the dry skin and the coefficient of static friction ($\mu$w) between the surface of the living-body contact electrode and the wet skin satisfy a relational expression below:

$$1.7 \leq (\mu w)/(\mu d) \leq 2.5$$

8. The living-body contact electrode according to any one of claims 5 to 7, wherein a water absorption rate of the stretchable conductor sheet is 0.5% by mass or more and 25% by mass or less.

9. A garment for measuring living-body information, the garment comprising a plurality of the living-body contact electrodes according to any one of claims 1 to 8.

10. The garment for measuring living-body information according to claim 9, wherein a coefficient of static friction ($\mu$d) between an electrical wiring portion surface excluding an electrode surface of a surface in contact with a living body of the garment for measuring living-body information and a dry skin is 0.5 or less.

11. The garment for measuring living-body information according to claim 9, wherein a coefficient of static friction ($\mu$d) between an electrical wiring portion surface excluding an electrode surface of a surface in contact with a living body of the garment for measuring living-body information and a wet skin is 0.4 or less.

12. The living-body contact electrode according to claim 1 or 2, wherein a conductive fabric is used as a conductive member on the surface of the living-body contact electrode.

13. The living-body contact electrode according to claim 3 or 4, wherein a conductive fabric is used as a conductive member on the surface of the living-body contact electrode.

14. The living-body contact electrode according to claim 12 or 13, wherein the conductive fabric is formed of a stretchable conductive cloth including at least a conductive fiber as a constituent element.

15. The living-body contact electrode according to any one of claims 12 to 14, wherein the conductive fabric is at least one conductive fabric selected from a woven fabric, a knitted fabric, or a nonwoven fabric including a metal-covered synthetic fiber at 10% by mass or more.

16. The living-body contact electrode according to any one of claims 12 to 14, wherein the conductive fabric includes a hybrid material of a chemically synthesized fiber and a conductive polymer.

17. The living-body contact electrode according to any one of claims 12 to 16, wherein the coefficient of static friction $(\mu d)$ between the surface of the living-body contact electrode and the dry skin and the coefficient of static friction $(\mu w)$ between the surface of the living-body contact electrode and the wet skin satisfy a relational expression below:

$$0.8 \leq (\mu w)/(\mu d) \leq 1.5$$

18. The living-body contact electrode according to any one of claims 12 to 17, wherein a water retention of the conductive fabric is 40% by mass or more and 350% or less.

19. A garment for measuring living-body information, the garment comprising a plurality of the living-body contact electrodes according to any one of claims 12 to 18.

20. The garment for measuring living-body information according to claim 19, wherein a coefficient of static friction $(\mu d)$ between an electrical wiring portion surface excluding an electrode surface of a surface in contact with a living body of the garment for measuring living-body information and a dry skin is 0.5 or less.

21. The garment for measuring living-body information according to claim 19, wherein a coefficient of static friction $(\mu d)$ between an electrical wiring portion surface excluding an electrode surface of a surface in contact with a living body of the garment for measuring living-body information and a wet skin is 0.4 or less.

# EP 3 677 176 A1

**INTERNATIONAL SEARCH REPORT**

| International application No. |
| --- |
| PCT/JP2018/031121 |

**A. CLASSIFICATION OF SUBJECT MATTER**
Int.Cl. A61B5/0408(2006.01)i

According to International Patent Classification (IPC) or to both national classification and IPC

**B. FIELDS SEARCHED**

Minimum documentation searched (classification system followed by classification symbols)
Int.Cl. A61B5/04, H01B1/00, A41D13/00

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2017-35457 A (TOYOBO CO., LTD.) 16 February 2017, paragraphs [0066], [0072]-[0088], fig. 4 (Family: none) | 1-21 |
| A | JP 2016-36642 A (NIPPON TELEGRAPH AND TELEPHONE CORP.) 22 March 2016, entire text, all drawings (Family: none) | 1-21 |

☒ Further documents are listed in the continuation of Box C.     ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 November 2018 (08.11.2018) | 20 November 2018 (20.11.2018) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2018/031121

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2015-93167 A (NITTO DENKO CORP.) 18 May 2015, entire text, all drawings (Family: none) | 1-21 |
| A | JP 2016-182755 A (SUMINOE TEXTILE CO., LTD.) 20 October 2016, entire text, all drawings (Family: none) | 1-21 |
| A | JP 2013-16473 A (SEKISUI CHEMICAL CO., LTD.) 24 January 2013, entire text, all drawings (Family: none) | 1-21 |
| A | JP 2000-345010 A (MITSUI CHEMICALS, INC.) 12 December 2000, entire text, all drawings & US 6812065 B1, entire text, all drawings & CN 1310848 A & KR 10-0402154 B1 | 1-21 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9215668 A
- JP 11225977 A
- JP 4860155 B
- JP 2016036642 A
- JP 2016182755 A